# EUROPÄISCHE PATENTANMELDUNG

(11) **EP 2 606 904 A1**
(43) Veröffentlichungstag der Anmeldung: **26.06.2013**
(21) Anmeldenummer: 11010116.9
(22) Anmeldetag: 22.12.2011
(51) Int. Cl.: A61K 38/01, A61K 31/401, A61K 31/7008, A61K 31/737, A61P 19/02

(54) **Arznei- oder Nahrungsergänzungsmittel zur Behandlung und/oder Prophylaxe von Gelenkerkrankungen**

(71) Anmelder: Alber, Axel, 69120 Heidelberg (DE)
(72) Erfinder: Alber, Axel, 69120 Heidelberg (DE)
(74) Vertreter: Lobemeier, Martin Landolf

(57) **Zusammenfassung**

Arznei- oder Nahrungsergänzungsmittel zur Behandlung und/oder Prophylaxe von Gelenkerkrankungen, mit einem Kollagenhydrolysat, gekennzeichnet durch freies Hydroxyprolin.

## Beschreibung

Die Erfindung betrifft ein Arznei- oder Nahrungsergänzungsmittel zur Behandlung und/oder Prophylaxe von Erkrankungen der Gelenke, insbesondere Arthrose, die ein Kollagenhydrolysat aufweisen.

Als Kollagenhydrolysat wird enzymatisch und/oder thermisch hydrolisiertes Kollagen bezeichnet, das als Kollagenhydrolysat gut dispergierbar und emulsionsstabilisierend ist. Neben dem Einsatz von Kollagenhydrolysat in der kosmetischen, in der Lebensmittel- und in der pharmazeutischen Industrie, beispielsweise als Tablettiergsgsmittel, kommt dem Kollagenhydrolysat selbst auch eine Rolle als Arznei- oder Nahrungsergänzungsmittel zu.

Insbesondere wird die perorale Einnahme von Kollagenhydrolysat bei Gelenkerkrankungen, speziell Arthrose, als therapeutische Maßnahme oder auch generell als prophylaktische Maßnahme empfohlen. Dabei soll die Knorpelneubildung durch die Bereitstellung der dafür benötigten Bausteine angeregt werden.

Degenerative Gelenkerkrankungen zeichnen sich nämlich insbesondere durch einen erhöhten Abbau von Knorpelgewebe, einer verstärkten Degradation von Matrixmakromolekülen bei gleichzeitig verringerter Synthese von Kollagen und Proteoglykanen aus. Der dadurch bedingte progressive Verlust an Knorpelsubstanz und die daraus resultierenden entzündlichen Reaktionen entstehen für an derartigen Gelenkerkrankungen leidenden Patienten starke Gelenkschmerzen, die zur Vermeidung der Schmerzen auch zu einer verringerten Bewegungsaktivität bzw. zur Einnahme starker Schmerzmittel führen.

Die perorale Einnahme von Kollagenhydrolysat führt zu einer erhöhten Kollagensyntheseaktivität und stellt zugleich die zur Kollagensynthese benötigten Bausteine bereit, sodass einer weiteren Degeneration der Gelenke entgegengewirkt werden und die Situtation des Patienten verbessert werden kann.

Allerdings ist die Wirkung von Kollagenhydrolysat allein nicht in allen Fällen befriedigend, sodass es Aufgabe der Erfindung ist, auf der Basis von Kollagenhydrolysat ein Arznei- oder Nahrungsergänzungsmittel zur Behandlung und/oder Prophylaxe von Erkrankungen der Gelenke, insbesondere Arthrose, mit höherer Wirkung zu schaffen

Diese Aufgabe wird durch das Arznei- oder Nahrungsergänzungsmittel mit den Merkmalen von Anspruch 1 gelöst. Die Unteransprüche geben vorteilhafte Ausgestaltungen der Erfindung an.

Grundgedanke der Erfindung ist es, ein peroral zu verabreichendes Arznei- oder Nahrungsergänzungsmittel anzubieten, das neben Kollagenhydrolysat auch freies Hydroxyprolin enthält. Es hat sich nämlich herausgestellt, dass die Wirkung des Arzneimittels bzw. Nahrungsergänzungsmittels erheblich verbessert werden kann, wenn zusätzlich zu dem mit dem Kollagenhydrolysat bereitgestellten Hydroxyprolin auch freies Hydroxyprolin im Arzneimittel bzw. Nahrungsergänzungsmittel enthalten ist.

Bevorzugt besitzt das zur Herstellung des Arznei- oder Nahrungsergänzungsmittels verwendete Kollagenhydrolysat ein Molekulargewicht von kleiner 3,1 kD und einen Hydroxyprolingehalt von 11-12 %. Dabei ist das Kollagenhydrolysat aus Kollagen vom Typ I, II und/oder III hergestellt.

Bevorzugt weist das Arznei- oder Nahrungsergänzungsmittel auch einen freien Aminozucker, bevorzugt Glucosamin, oder ein Salz eines Aminozuckers, bevorzugt Glucosaminchlorid oder Glucosaminsulfat, auf.

Alternativ oder zusätzlich weist das Arznei- oder Nahrungsergänzungsmittel auch Chondroitin oder ein Chondroitinsalz, bevorzugt Chondroitinsulfat, auf.

Eine vorteilhafte Zusammensetzung des Arznei- oder Nahrungsergänzungsmittels weist 60-92 Gew.-% Kollagenhydrolysat, 1-10 Gew.-% freies Hydroxyprolin, 5-20 Gew.-% Glucosamin und 2-10 Gew.-% Chondroitin, jedoch bevorzugt 80-92 Gew.-% Kollagenhydrolysat, 1-5 Gew.-% freies Hydroxyprolin, 5-10 Gew.-% Glucosamin und 2-5 Gew.-% Chondroitin auf.

Besonders bevorzugt ist eine Formulierung des Arznei- oder Nahrungsefgänzungsmittels folgender Zusammensetzung:

| Substanz | Gew.-% | Anmerkungen |
|---|---|---|
| Kollagenhydrolysat | 84,8 | MW<3,1 kD, 11-12% Hydroxyprolin |
| Hydroxyprolin | 2,7 | --- |
| Glucosamin | 8,3 | Gluc-HCl, Gluc-SO₄ |
| Chondroitin | 4,2 | Chon-SO₄ |

Neben den in dieser besonders bevorzugten Formulierung genannten Bestandteile können auch deren Salze verwendet werden.

Für die in der Tabelle vorstehend genannte besonders bevorzugte Formulierung hat sich beim Menschen mit einem Körpergewicht von 75 kg eine tägliche Einnahme von 3 g als besonders vorteilhaft erwiesen. Bei Katzen liegt die vorteilhafte tägliche Gabe bei 0,5 g, bei Hunden bei 0,5 g bis 4,0 g und bei Pferden zwischen 8 und 50 g.

Liegt bereits eine akute Gelenkerkrankung vor sollte die tägliche Gabe beim Menschen auf 6 g, bei der Katze auf 1 g, bei Hunden auf 1 g bis 8 g und bei Pferden auf 16 g bis 100 g, also um das Doppelte, erhöht werden.

Selbstverständlich können auch andere (Säuge-) Tiere mit dem erfindungsgemäßen Arznei-oder Nahrungsergänzungsmittel behandelt werden. Die vorstehenden Werte sind daher als (vom Körpergewicht oder von anderen physiologischen Parametern abhängige) Richtwerte zu verstehen.

Insbesondere bewirkt die tägliche perorale Aufnahme der erfindungsgemäßen Formulierung innerhalb von drei Monaten einen signifikanten Rückgang der mit Gelenkerkrankungen assoziierten Schmerzen, einen damit einhergehenden geringeren Bedarf an Schmerzmitteln und eine bessere Beweglichkeit der so behandelten Patienten.

Nach täglicher Aufnahme des erfindungsgemäßen Arznei- oder Nahrungsergänzungsmittels kann eine Stimulation der Produktion von Knorpelgewebe, eine Reduktion der Entzündungsreaktionen sowie des Knorpelabbaus beobachtet werden. Bei längerer Anwendung über drei Monate hinaus resultiert die Anwendung in glatten, geschmeidigen Knorpelgewebeoberflächen, die zur Ausübung einer beschwerdefreien Gelenkfunktion notwendig sind.

## Patentansprüche

1. Arznei- oder Nahrungsergänzungsmittel zur Behandlung und/oder Prophylaxe von Gelenkerkrankungen, mit einem Kollagenhydrolysat,
**gekennzeichnet durch**
freies Hydroxyprolin.

2. Arznei- oder Nahrungsergänzungsmittel nach Anspruch 1, **gekennzeichnet durch** einen freien Aminozucker oder ein Salz davon.

3. Arznei- oder Nahrungsergänzungsmittel nach einem der vorhergehenden Ansprüche, **gekennzeichnet durch** Chondroitin oder ein Salz davon.

4. Arznei- oder Nahrungsergänzungsmittel nach einem der vorhergehenden Ansprüche, **dadurch gekennzeichnet, dass** das Kollagenhydrolysat ein Molekulargewicht von kleiner 3,1 kD und einen Hydroxyprolingehalt von 11-12 % aufweist und aus Kollagen vom Typ I, II und/oder III hergestellt ist.

5. Arznei- oder Nahrungsergänzungsmittel nach einem der vorhergehenden Ansprüche, **gekennzeichnet durch** die Zusammensetzung von 60-92 Gew.-% Kollagenhydrolysat, 1-10 Gew.-% Hydroxyprolin, 5-20 Gew.-% Glucosamin und 2-10 Gew.-% Chondroitin.

6. Arznei-- oder Nahrungsergänzungsmittel nach einem der vorhergehenden Ansprüche, **gekennzeichnet durch** die Zusammensetzung von 80-92 Gew.-% Kollagenhydrolysat, 1-5 Gew.-% Hydroxyprolin, 5-10 Gew.-% Glucosamin und 2-5 Gew.-% Chondroitin.

7. Arznei- oder Nahrungsergänzungsmittel nach einem der vorhergehenden Ansprüche, **gekennzeichnet durch** die Zusammensetzung von 84,8 Gew.-% Kollagenhydzolysat, 2,7 Gew.-% Hydroxyprolin, 8,3 Gew.-% Glucosamin und 4,2 Gew.-% Chondroitin.
